# EUROPEAN PATENT SPECIFICATION

(11) **EP 3 048 961 B1**
(45) Date of publication and mention of the grant of the patent: **25.01.2017**
(21) Application number: 15739571.6
(22) Date of filing: 17.07.2015
(51) Int. Cl.: A61B 5/024

(54) **HEART RATE MONITOR SYSTEM AND METHOD OF DETERMINING A WARMING-UP STATUS OF A USER**
HERZFREQUENZÜBERWACHUNGSSYSTEM UND VERFAHREN ZUR BESTIMMUNG EINES AUFWÄRMSTATUS EINES BENUTZERS
SYSTÈME DE SURVEILLANCE DE RYTHME CARDIAQUE ET PROCÉDÉ PERMETTANT DE DÉTERMINER LE STATUT D'ÉCHAUFFEMENT D'UN UTILISATEUR

(30) Priority: 28.07.2014 EP 14178696
(43) Date of publication of application: 03.08.2016
(73) Proprietor: Koninklijke Philips N.V., 5656 AE Eindhoven (NL)
(72) Inventor: GELISSEN, Jozef Hubertus, NL-5656 AE Eindhoven (NL); HUIJBREGTS, Laurentia Johanna, NL-5656 AE Eindhoven (NL)
(74) Representative: Roche, Denis
(86) International application number: PCT/EP2015/066370
(87) International publication number: WO 2016/016016

(56) References cited:
- US-A1- 2012 035 021
- US-A1- 2014 073 486
- US-A1- 2014 127 996
- English Contents: "RS800CX User Manual", , 31 December 2013 (2013-12-31), XP055143064, Retrieved from the Internet: URL:http://www.polar.fi/e_manuals/RS800CX/ Polar_RS800CX_user_manual_English/manual.p df [retrieved on 2014-09-26]
- "Development and Evaluation of Polar OwnZone Feature", , 31 May 2002 (2002-05-31), XP055143236, Retrieved from the Internet: URL:http://www.cardiozonepr.com/images/own zone_whitepaper.pdf [retrieved on 2014-09-29]

## Description

### FIELD OF THE INVENTION

The invention relates to a heart rate monitor system as well as a method of determining a warming-up status of a user.

### BACKGROUND OF THE INVENTION

Optical heart rate sensors are well known to monitor or detect a heart rate of a user. Such a heart rate sensor can be based on a photoplethysmograph (PPG) sensor and can be used to acquire a volumetric organ measurement. By means of pulse oximeters, changes in light absorption of a human skin is detected and based on these measurements, a heart rate of a user can be determined.

US 2014/0073486 A1 shows a wearable physiological measurement system which uses a PPG signal to determine the heart rate of a user. The heart rate or the heart rate variability is determined and, based on these measurements, it is determined whether a user should warm-up for a longer period of time or is still in his/her warm-up stage.

Typically, a transmissive or reflective blood PPG sensor monitors the perfusion of blood to the dermis and subcutaneous tissue of the skin through absorption measurements at a specific wavelength. The PPG signals can comprise a small AC signal (the actual photoplethysmogram) on top of a large unwanted DC offset signal. The DC offset signal can comprise signals which may originate from the skin or tissue as well as from a considerable part of ambient light.

### SUMMARY OF THE INVENTION

It is an object of the invention to provide a heart rate monitor system which is able to detect a warming-up status of a user as well as a corresponding method for determining a warming-up status of a user.

In an aspect of the present invention, a heart rate monitor system is provided. The heart rate monitor system comprises an optical heart rate sensor configured to measure or determine a heart rate of a user and to output an output signal. The heart rate monitor system furthermore comprises an analyzing unit configured to analyze an output signal from the at least one heart rate sensor and to determine a warming-up status of a user based on the analyzed output signal from the optical heart rate sensor. The analyzing unit comprises a signal analyzer configured to analyze at least one of an AC component of the output signal, a DC component of the output signal and a pulse morphology of the output signal. The analyzing unit furthermore comprises a warm-up detector unit configured to detect a warming-up status of the user based on blood perfusion of the user as detected according to signal characteristics of the AC component, the DC component or the pulse morphology of the output signal from the optical heart rate sensor. The heart rate monitor system is thus able to determine a warming-up status of a user based on measured physiological signals as determined by the optical heart rate sensor such that the detection of a warming-up status is performed more accurately.

In a further aspect of the invention, the optical heart rate sensor comprises at least one light source configured to generate light, which is directed towards a skin of a user. The optical heart rate sensor furthermore comprises at least one photo detector unit configured to detect light which is indicative of an absorption or reflection of the light from the at least one light source in or from the skin of a user. The output signal of the optical heart rate sensor corresponds to output signals of the at least one photo detector unit. Accordingly, the output of the photo detector unit is analyzed by the analyzing unit and is thereby used to determine the warming-up status of the user.

According to still a further aspect of the invention, the signal characteristics can include an amplitude of the AC component, the DC component of the output signal or a history of the output signal (like the increase/decrease of the amplitude) or the pulse morphology of the output signal. These signal characteristics can be instant values or can be time averages.

According to still a further aspect of the invention, the warm-up detector unit is configured to compare the analyzed signal characteristic and/or their absolute and/or relative changes over time to threshold values to determine the warming-up status of the user. When e.g. the amplitude does not show a (significant) increase, optimal perfusion has been reached. According to still a further aspect of the invention, the heart rate monitor system comprises an output unit configured to output the warming-up status of the user.

According to still a further aspect of the invention, a method of determining a warming-up status of the user is provided. A heart rate of a user is measured or determined by means of an optical heart rate sensor and the output signal is outputted. The output signal from the at least one optical heart rate sensor is analyzed and a warming-up status of a user is determined based on the analyzed output signal from the optical heart rate sensor.

According to an aspect of the invention, a blood perfusion and thereby a warming-up status of a user is detected by using an optical heart rate sensor and by analyzing the output signals of the optical heart rate sensor. In particular, the blood perfusion has an influence on the output signal, for example the signal-to-noise ratio, of the optical heart rate sensor.

According to an aspect of the invention, a computer program product comprising a computer readable memory storing computer program code means for causing the above heart rate monitor system to carry out the steps of the method of determining a warming-up status of a user is provided.

It shall be understood that the heart rate monitor system of claim 1, the method of determining a warming-up status of a user of claim 7 and the computer program product of claim 8, have similar and/or identical preferred embodiments, in particular, as defined in the dependent claims.

It shall be understood that a preferred embodiment of the present invention can also be a combination of the dependent claims or above embodiments or aspects with respective independent claims.

These and other aspects of the invention will be apparent from and elucidated with reference to the embodiment(s) described hereinafter.

### BRIEF DESCRIPTION OF THE DRAWINGS

In the following drawings:
Fig. 1 shows a basic block diagram of a heart rate monitor system according to an aspect of the invention,
Fig. 2 shows a graph depicting an output signal from a heart rate sensor according to an aspect of the invention,
Fig 3 shows a graph indicating an output signal of the heart rate sensor without its AC component as well as movement data of a user according to an aspect of the invention,
Fig. 4 shows a graph indicating an output signal of the heart rate sensor with its AC component as well as movement data,
Fig. 5 shows a graph indicating an AC component of an output signal of a heart rate sensor as well as movement data according to a further aspect of the invention,
Fig. 6 shows a graph indicating an output signal of the heart rate sensor as well as a modulation of the output sensor according to an aspect of the invention,
Fig. 7 shows a graph indicating the output signals of a heart rate sensor as well as a movement data of a user according to an aspect of the invention,
Fig. 8 shows a graph indicating an output signal of a heart rate sensor as well as a modulation of the output signal over 20 intervals measured on a wrist of a user,
Fig. 9 shows a graph indicating an output signal of the heart rate sensor as well as the modulation of the output signals over 17 intervals measured on a calf of a user, and
Fig. 10 shows a graph indicating output signals of an optical heart rate sensor using red light and infrared light according to an aspect of the invention.

### DETAILED DESCRIPTION OF EMBODIMENTS

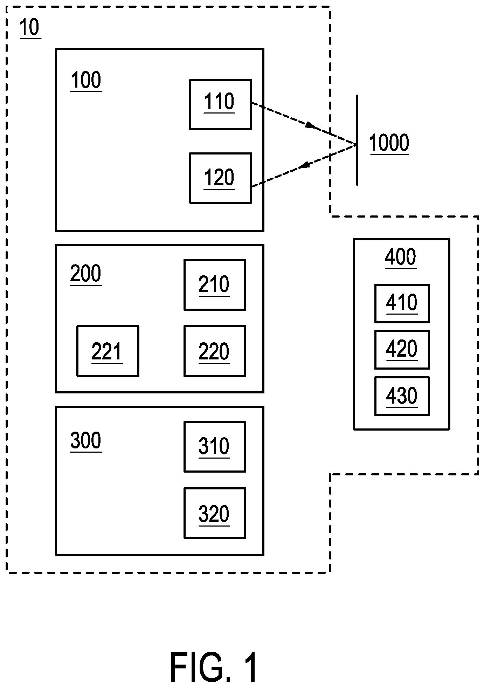

Fig. 1 shows a schematic block diagram of a heart rate monitor system according to an aspect of the invention. The heart rate monitor system 10 comprises at least one heart rate sensor 100, an analyzing unit 200, optionally an output unit 300 and optionally at least one secondary sensor 400. The heart rate sensor 100 comprises at least one light source 110 and at least one photo detector 120. Several photo detectors can be combined into a photo detector unit. The photo detector unit may also comprise only one photo detector. The light source 110 emits light onto a skin 1000 of a user and the photo detector 120 detects reflected or transmitted light from or through the skin 1000 of the user. The light source 110 can be embodied as LEDs or laser. The heart rate sensor 100 can thus be implemented as an optical heart rate sensor. The heart rate sensor 100 can be operated in a transmittance mode (measuring light transmitted through the skin 1000 of a user) or in a reflectance mode (measuring the light reflected from the skin 1000 of the user).

In an aspect of the invention, the optical heart rate sensor 100 is implemented as a photoplethysmography (PPG) sensor.

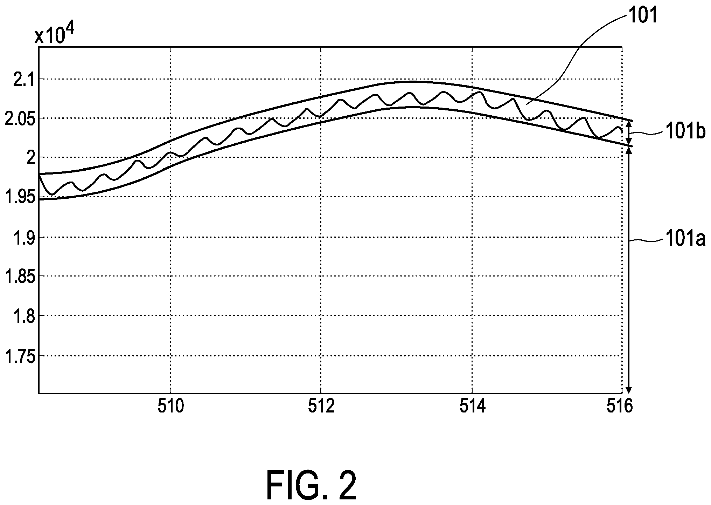

The photo detector 120 detects light reflected or transmitted from the skin 1000 of the user. In fact, the heart rate sensor 100 optically measures the variation in blood volume in the human tissue and can thus detect a pulse signal based on these measurements. The output signal of the at least one photo detector 120 can be the output signal 101 of the heart rate sensor 100. The output signal of the hear rate sensor 100 can have a DC component and a AC component. This will be described in more detail with reference to Fig. 2.

The analyzing unit 200 can comprise a signal analyzer 210 and a warm-up
detector 220. The warm-up detector 220 can optionally be coupled to a memory 221 in which threshold values can be stored. Alternatively or in addition, the warm-up detector 220 can also detect that the warming-up is completed by calculating the changes of the amplitudes over time. If there is no (significant) change of the amplitude anymore, the user is warmed-up and a good perfusion is achieved.

The output unit 300 can optionally have a graphic user interface 310 as well as optionally a wireless interface 320. Via the wireless interface 320, the heart rate monitor system can wirelessly communicate e.g. with a smartphone, tablet or laptop.

The secondary sensor unit 400 can comprise at least one secondary sensor like an accelerometer 410 for measuring movement of a user, a temperature sensor 420 and/or a humidity sensor 430. The data from the secondary sensor unit 400 can be used to validate the output of the warm-up detector 220.

According to an aspect of the invention, the signal analyzer 210 analyzes the output signal 101 of the optical heart rate sensor 100. In particular, the signal analyzer 210 can analyze a DC component, an AC component and/or the pulse morphology of the output signal 101. In particular, the morphology of the pulses in the PPG signal can be analyzed. Furthermore, the signal analyzer 210 can also analyze the variations in the DC component, the AC component and/or the pulse morphology of the output signal 101. The warm-up detector 220 determines a warming-up status of a user by analyzing the DC component, the AC component and/or the pulse morphology of the output signal 101 of the optical heart rate sensor 100. In other words, the warm-up detector 220 detects a warming-up status of the user based on signal characteristics of the output signal (like the amplitude of the AC component and/or the history of the output signal of the PPG sensor). Optionally, the warm-up detector 220 can compare the analyzed output signal 101 with threshold values which are for example stored in the memory 121. Based on these comparisons, the warm-up detector 220 can determine the warming-up status of a user.

The warm-up detector 220 may also monitor changes of the amplitude of the output signal from the sensor 100. If no (significant) changes are detected, the warm-up detector may determine that the warm-up is completed.

A warming-up is typically performed before exercises like running, playing soccer or the like. A warming-up is typically done by physical activity of lower intensity. In particular, the muscles are moved. The aim can be to increase the body temperature, to increase the activity of the cardio vascular system, to increase the perfusion. The warming-up is useful to decrease the risk of injuries. Through the warming-up, the perfusion is increased. This can lead to an increased muscular activity. Accordingly, more blood flows through the muscles and thus the muscles can be supplied with oxygen.

Fig. 2 shows a graph indicating an output signal of a heart rate sensor according to an aspect of the invention. The heart rate sensor according to Fig. 2 may be based on the heart rate sensor according to Fig. 1. In particular, the output signal 101 of the heart rate sensor comprises a DC component 101a as well as an AC component 101b. The output signal 101 also has a pulse morphology. In Fig. 2, a typical signal from a PPG sensor worn at the wrist is depicted. The DC component 101a of the output signal 100 does not contain heart rate information. However, the AC component 101b is the signal from which the heart rate can be derived. The ratio of the AC component to the DC component is the modulation and is an indication of the amount of useful signals for the heart rate derivation. The amplitude of the heart rate signal is the height of the AC component.

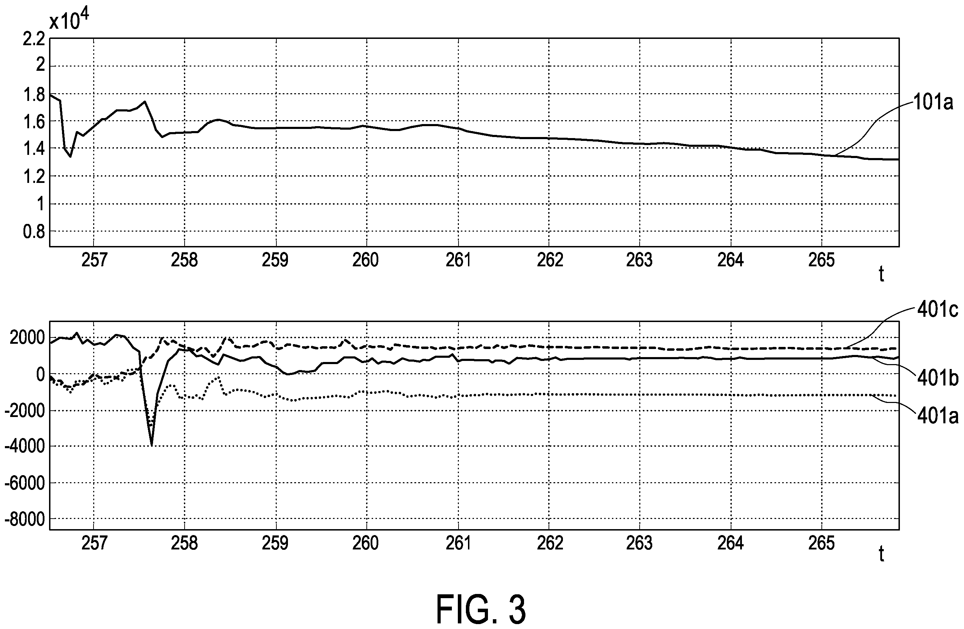

Fig. 3 shows a graph indicating an output signal from the heart rate sensor as well as movement data. In particular, in Fig. 3, the output signal 101 of the optical rate sensor is depicted. Furthermore, corresponding movement data 401 a, 401 b, 401c is also depicted. As can be seen from the depicted output signal of the heart rate sensor, only a DC component 101a but not an AC component 101b is present although the user is not moving as can be seen by the movement data 401a - 401c. Accordingly, the heart rate sensor 100 is not able to detect a pulse signal.

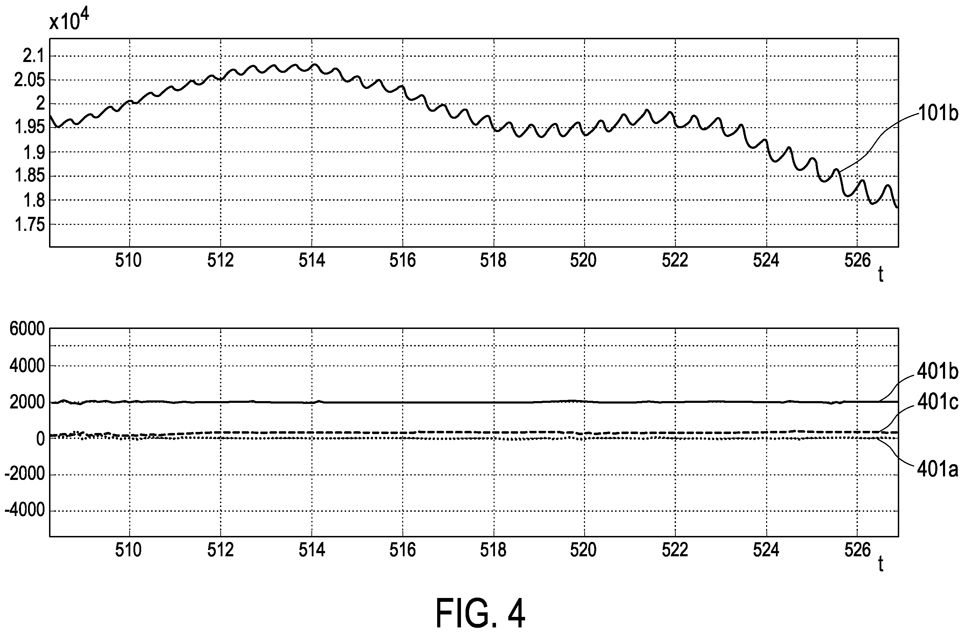

Fig. 4 shows a graph indicating an output signal of a heart rate sensor as well as movement data according to an aspect of the invention. Measurements as depicted in Fig. 4 are measured at a later point of time as compared to the measurements of Fig. 3. Here, it can be seen from the graph below that the user is not moving and that an AC component 101b is detectable such that a pulse signal can be derived. The wrinkle in the output signal corresponds to the changes in reflection which are caused by heart beats. The amplitude of these wrinkles can change in accordance with the activity of the user. This can in particular happen during the first minutes during a warm-up period.

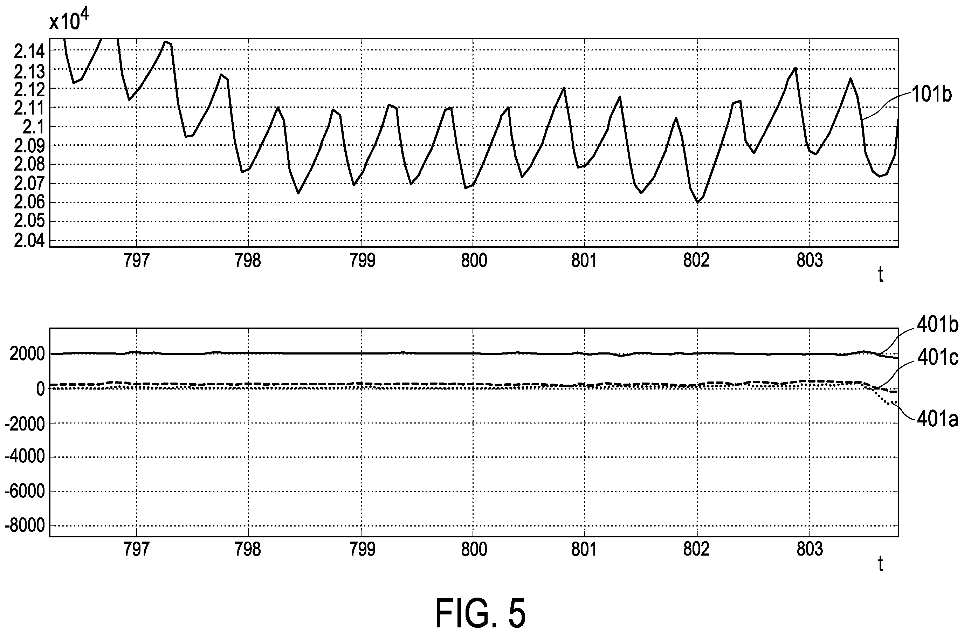

Fig. 5 shows a graph indicating an output signal of a heart rate sensor as well as movement data. The measurements according to Fig. 5 have been measured at a later point of time as compared to Fig. 4. The amplitude of the AC component 101b increases from Fig. 3 (where it is basically zero) to Fig. 4 (with an amplitude of 0,03 x 10⁴) to an amplitude of 0,04 x 10⁴.

The Figs. 3 - 5 show that when taking a small pause during the run (as indicated by a flat line in the accelerometer data), the amplitude of the PPG output signal can increase due to a better perfusion (the runner is warmed up). During this pause, the PPG signal will not be disturbed by motion artifacts.

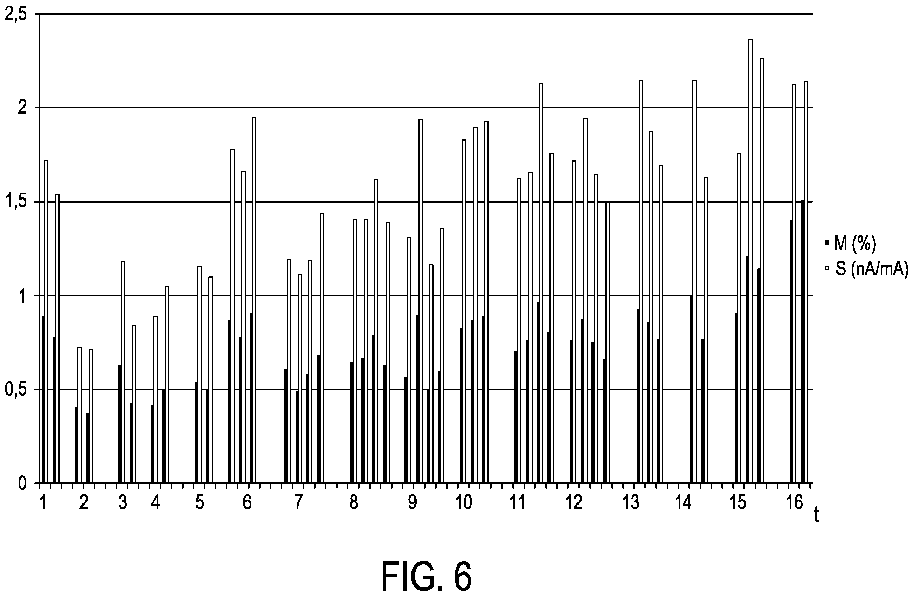

Fig. 6 shows a graph indicating an amplitude S of the output signal as well as the modulation M of the output signal. The modulation M is depicted in % and the amplitude S in nA/mA. At t = 1, the user is inside a warm environment. At t = 2, the user has run 1 kilometer and is sitting down on a bench and has a relative low perfusion. At t = 3 - 14, the user is running stairs up and down and then sitting on a bench until the heart rate drops back to 120 bpm. Here, it can be seen that the perfusion starts to increase. At t = 15, a four kilometer run is performed followed by sitting down on a bench until the heart rate drops back to 120 bpm. At t = 16, a final 2 kilometer run is performed and the user is arriving back at home and is sitting on a chair until the heart rate drops below 90 bpm. As can be seen from Fig. 6, the modulation M and the signal S changes over time reacting to the different stages the body goes through during the exercise. At t = 2, the modulation M and the signal amplitude S decreases as the body tries to preserve body heat. Thereafter, the body starts to slowly warm up, for example at t = 10 where the amplitude increases. In Fig. 6, the warm-up period has been extended due to the relative short exercises and long idle stages. At t = 6, the user has performed two runs immediately after each other and thus the amplitude of the output signal increases.

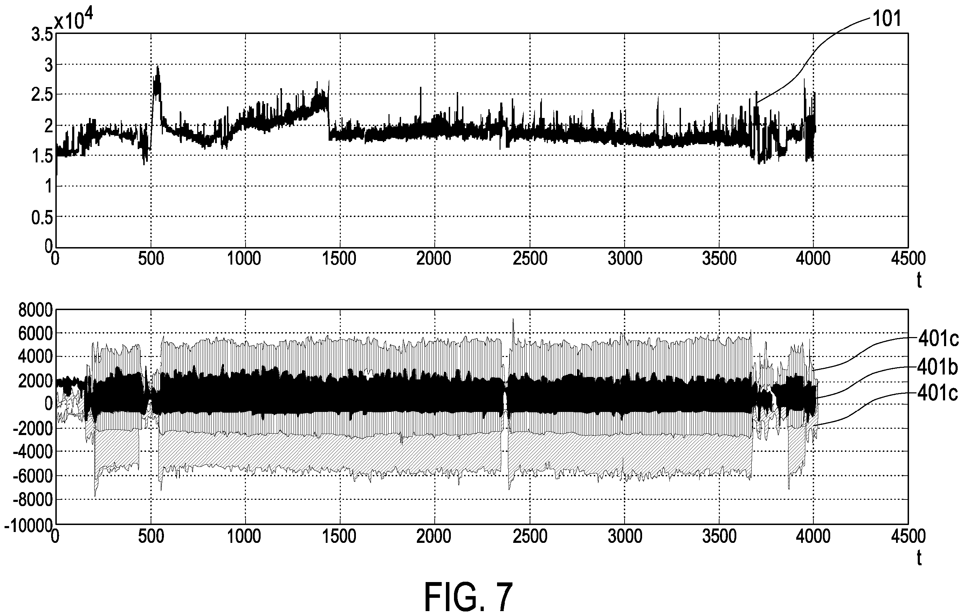

Fig. 7 shows a graph indicating an output signal of a heart rate sensor as well as output signals of motion sensors according to an aspect of the invention. In Fig. 7, the exercise takes 4.000 seconds and in the upper part, the output signal 101 of the heart rate sensor is depicted, while in the lower part, the motion data from the user is depicted. During t = 220 - 3.700 s, the acceleration is not changing very much except for interruptions at t = 500 and t = 2.400, when the user is standing idle. As can be seen from Fig. 7, the amplitude of the output signal 101 increases from the start (t = 295 s) and the middle (t = 2.096 s).

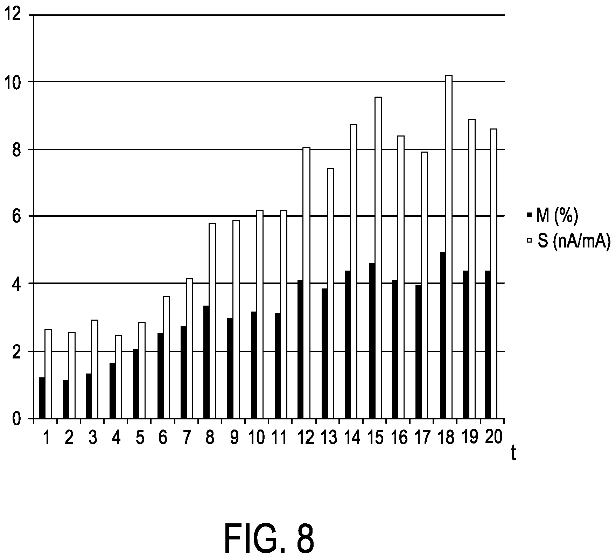

Fig. 8 shows a graph indicating a signal S and a modulation M of the output signal 101 according to an aspect of the invention as seen for several intervals 1 - 20, wherein the intervals can be 10 seconds each. In particular, the optical heart rate sensor 100 is attached to a wrist of a user. The graph of Fig. 8 relates to the graph of Fig. 7.

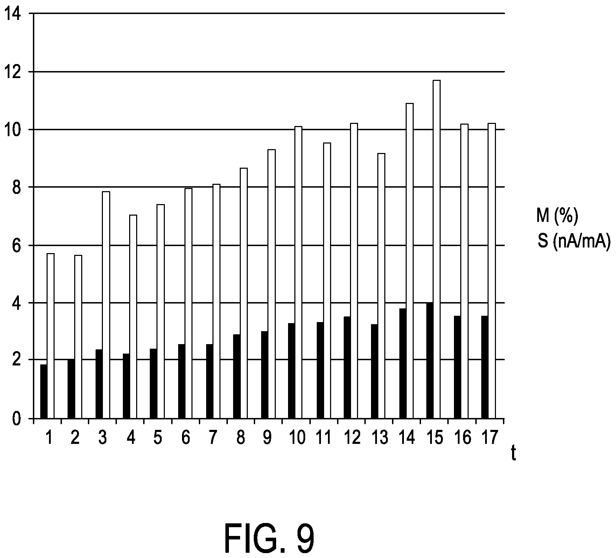

Fig. 9 shows a graph indicating a signal and a modulation of the output signal 101 of the heart rate sensor according to an aspect of the invention during the same exercise as shown in Fig. 7, wherein the heart rate sensor is attached to a calf of a user. By comparing the Figs. 8 and 9, it can be seen that the output signal increases more for a heart rate sensor attached to the wrist than for a heart rate sensor attached to the calf of the user, although the arms are not directly involved in the propulsion of the user.

It should be noted that the increased DC component as well as the increased modulation of the output signal 101 of the heart rate sensor can be explained as the blood perfusion increases during warm-up stages.

During a run with a relative constant movement, the amplitude of the PPG signal can increase during rest as long as movement artifacts are relatively constant.

It should be noted that if a user is doing a warm-up with different kinds of exercises like side shuffle, carioca, high knees, butt kicks etc., it may be difficult to measure an increase of perfusion if the warm-up exercise is relatively short and if there are rest periods in between the exercises, it might be better to use those. On the other hand, if the exercise has a returning pattern, the heart rate monitor system may be able to perform some activity classification (for example based on accelerometer data) to enable an effective detection of a warm-up status even during the exercises.

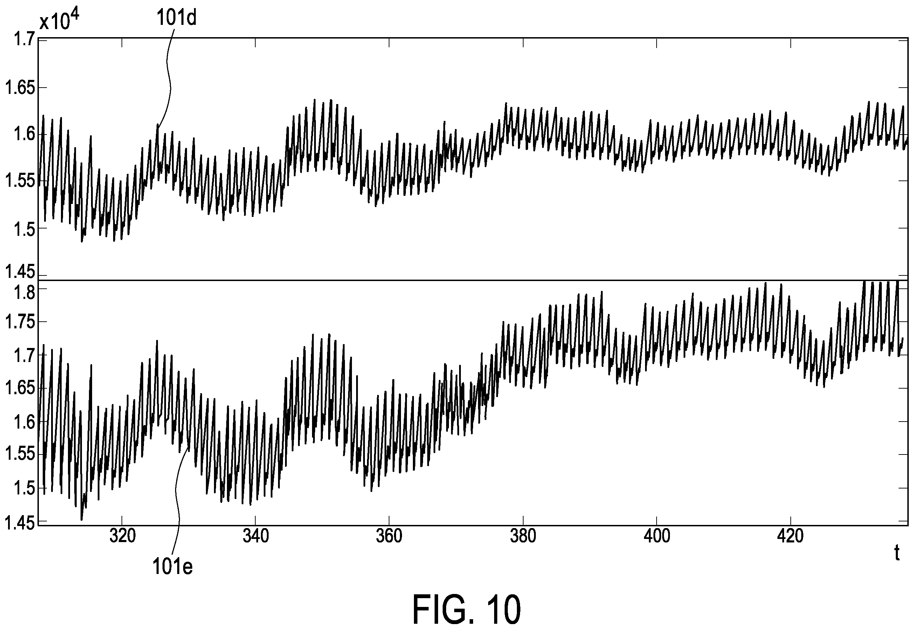

Fig. 10 shows a graph indicating output signals of a heart rate sensor according to the invention placed at a finger tip. In the upper part of Fig. 10, the output signal 101d of an optical heart rate sensor using red light and in the lower part the output signal 101e of an optical heart rate sensor using infrared is depicted. Furthermore, in Fig. 10, a change in vasomotor tone is depicted, which was induced at t = 376 s by submerging the hand without sensor in cold water, It should be noted that the vasoconstriction can be present through the whole body thus leading to vasoconstriction. As can be seen from Fig. 10, the modulation, as well as the pulse morphology of the output signal, changes.

Accordingly, the heart rate monitor system according to the invention analyzes the output signal of the heart rate sensor to derive a warming-up status of a user. As the warming-up status of a user is directly connected to the blood perfusion, detecting a blood perfusion makes it possible to determine the warming-up status. The heart rate monitor system according to the invention uses the relationship of a low signal-to-noise ratio (low modulation) of the output signal 101 of the heart rate sensor to determine the blood perfusion. In other words, if the signal-to-noise ratio of the output signal of the heart rate sensor 100 is good, then also the blood perfusion is good and the user is warmed up. On the other hand, if the signal-to-noise ratio is low, then the blood perfusion is also low and the user is not warmed up correctly. If a user is starting an exercise in a cold environment, the human body will try to preserve body heat by closing the capillaries thus by starting a vasoconstriction.

According to an aspect of the invention, a warming-up status of a user can be detected by actually measured physiological signals of the user. Thus, a very effective and accurate determination of the warming-up status of the user can be performed.

According to an aspect of the invention, the signal analyzer 210 analyzes the AC component, the DC component or a combination of these like the modulation of the signal. Based on the signal analyzer 210, the warm-up detector 220 can detect a warming-up status of a user. For example, the warm-up detector can use an increase in the AC component, the DC component or the modulation of the output signal as well as the pulse morphology of the output signal 101 to determine the warm-up status of the user. The warm-up status of the user can be outputted by the output unit 300. This can be performed by the graphical user interface 310 or by the wireless interface 320. The user and/or his trainer can be informed of an efficient warming-up by optical or acoustical signals. Alternatively, a vibration signal can also be outputted by the output unit 300.

According to an aspect of the invention, the warm-up detector 220 can compare threshold values as stored in the memory 221 with the actual analyzed output signals of the heart rate sensor 100. As an example, a 5% increase in the DC component could be used as threshold value. In another aspect of the invention, the warm-up detector 220 can calculate the change in amplitude over time (e.g. by taking the average amplitude in the last 10 seconds and subtract the average amplitude in the 10 seconds before) and determine that the warm-up is sufficient when that value crosses a threshold of for example 5%. In addition or alternatively, the threshold values may also be user dependent or dependent on the circumstances of the exercise. The circumstantial threshold values may also take into account the outside temperature, the location of the sensor on the body and the activity data. User specific thresholds may also take into account the user's age, the gender, the ethnicity and/or skin tone and/or signal characteristics that were measured during previous exercises of the user.

The light sources 110 of the optical heart rate sensor 100 can be LEDs or lasers. Alternatively, the optical sensor can also be camera based and might operate using ambient light, without additional light source.

In a preferred embodiment of the invention, the heart rate monitor system can be embodied as a wrist device. Accordingly, a wrist device can comprise at least one optical heart rate sensor and an analyzing unit. Optionally, the wrist device can also comprise an output unit 300.

The heart rate monitor system can be embodied as a smart watch, as a wrist device, as smart glasses, smart bracelets or other wearable (smart) devices.

Other variations of the disclosed embodiment can be understood and effected by those skilled in the art in practicing the claimed invention from a study of the drawings, the disclosure and the appended claims.

In the claims, the word "comprising" does not exclude other elements or steps and in the indefinite article "a" or "an" does not exclude a plurality.

A single unit or device may fulfill the functions of several items recited in the claims. The mere fact that certain measures are recited in mutual different dependent claims does not indicate that a combination of these measurements cannot be used to advantage. A computer program may be stored/distributed on a suitable medium such as an optical storage medium or a solid state medium, supplied together with or as a part of other hardware, but may also be distributed in other forms such as via the internet or other wired or wireless telecommunication systems.

Any reference signs in the claims should not be construed as limiting the scope.

## Claims

1. Heart rate monitor system, comprising:
- an optical heart rate sensor (100), comprising:
- at least one light source (110) configured to generate light which is directed towards the skin (1000) of a user, and
- at least one photo detector unit (120) configured to detect light and output an output signal (101) which is indicative of an absorption or reflection of the light from the at least one light source (110) in or from the skin (1000);
- an analyzing unit (200) configured to determine a heart rate of a user based on the output signal (101) from the optical heart rate sensor (100);
**characterized in that**
- the analyzing unit (200) further comprises:
- a signal analyzer (210) configured to analyze at least one of an AC component, a DC component and pulse morphology of the output signal (101); and
- a warm-up detector unit (220) configured to determine a warm-up status of the user according to a measure of blood perfusion determined from characteristics of the AC component, the DC component or the pulse morphology of the output signal (101).

2. Heart rate monitor system according to claim 1, wherein the signal characteristics of the output signal (101) include an instant or time averaged value of the DC component, an instant or time averaged amplitude of the AC component, and/or an instant or time averaged pulse morphology.

3. Heart rate monitor system according to claim 2, wherein the warm-up
detector unit (220) is configured to compare the analyzed signal characteristics, their relative and/or absolute changes over time with threshold values to determine whether the user is already warmed-up.

4. Heart rate monitor system according to claim 3, further comprising an
output unit (300) configured to output the warm-up status of a user.

5. Method of determining a warm-up status of a user, comprising the steps of:
- generating light by a light source (110) and directing said light towards the skin (1000) of the user;
- detecting absorption or reflection of the light from the at least one light source (110) in or from the skin (1000) by a photo detector unit (120) and generating an output signal (101) indicative thereof;
- analyzing by a signal analyzer (210) at least one of an AC component, a DC component and pulse morphology of the output signal (101) and determining a heart rate of the user based on said output signal (101);
- determining by a warm-up detector unit (220) a warm-up status of the user according to a measure of blood perfusion determined from characteristics of the AC component, the DC component or the pulse morphology of the output signal (101).

6. A computer program product, comprising a computer readable memory storing computer program code means for causing the heart rate monitor system according to claim 1 to carry out steps of the method of determining a warm-up status of a user according to claim 5.

## Patentansprüche

1. Herzfrequenzüberwachungssystem, das Folgendes umfasst:
- einen optischen Herzfrequenzsensor (100), umfassend:
- mindestens eine Lichtquelle (110), die konfiguriert ist, um Licht zu erzeugen, das auf die Haut (1000) eines Benutzers gerichtet wird, und
- mindestens eine Fotodetektoreinheit (120), die konfiguriert ist, um Licht zu detektieren und ein Ausgangssignal (101) auszugeben, das eine Absorption oder Reflexion des Lichts von der mindestens einen Lichtquelle (110) in oder von der Haut (1000) angibt;
- eine Analyseeinheit (200), die konfiguriert ist, um eine Herzfrequenz eines Benutzers basierend auf dem Ausgangssignal (101) von dem optischen Herzfrequenzsensor (100) zu ermitteln;
**dadurch gekennzeichnet, dass**
- die Analyseeinheit (200) weiterhin Folgendes umfasst:
- einen Signalanalysator (210), der konfiguriert ist, um mindestens eines von einer AC-Komponente, einer DC-Komponente und einer Impulsmorphologie des Ausgangssignals (101) zu analysieren; und
- eine Aufwärmdetektoreinheit (220), die konfiguriert ist, um einen Aufwärmstatus des Benutzers entsprechend einem Maß der Blutperfusion zu ermitteln, die anhand von Eigenschaften der AC-Komponente, der DC-Komponente oder der Impulsmorphologie des Ausgangssignals (101) ermittelt wurde.

2. Herzfrequenzüberwachungssystem nach Anspruch 1, wobei die Signaleigenschaften des Ausgangssignals (101) einen momentanen oder zeitgemittelten Wert der DC-Komponente, einen momentanen oder zeitgemittelten Wert der AC-Komponente und/oder einen momentanen oder zeitgemittelten Wert der Impulsmorphologie umfassen.

3. Herzfrequenzüberwachungssystem nach Anspruch 2, wobei die Aufwärmdetektoreinheit (220) konfiguriert ist, um die analysierten Signaleigenschaften, ihre relativen und/oder absoluten Veränderungen über die Zeit mit Schwellenwerten zu vergleichen, um zu ermitteln, ob sich der Benutzer schon aufgewärmt hat.

4. Herzfrequenzüberwachungssystem nach Anspruch 3, weiterhin umfassend eine Ausgabeeinheit (300), die konfiguriert ist, um den Aufwärmstatus eines Benutzers auszugeben.

5. Verfahren zum Ermitteln eines Aufwärmstatus eines Benutzers, wobei das Verfahren die folgenden Schritte umfasst:
- Erzeugen von Licht durch eine Lichtquelle (110) und Richten des genannten Lichts auf die Haut (1000) des Benutzers;
- Detektieren der Absorption oder Reflexion des Lichts von der mindestens einen Lichtquelle (110) in oder von der Haut (1000) durch eine Fotodetektoreinheit (120) und Erzeugen eines diese angebenden Ausgangssignals (101);
- Analysieren, durch einen Signalanalysator (210), von mindestens einem von einer AC-Komponente, einer DC-Komponente und einer Impulsmorphologie des Ausgangssignals (101) und Ermitteln einer Herzfrequenz des Benutzers basierend auf dem genannten Ausgangssignal (101);
- Ermitteln, durch eine Aufwärmdetektoreinheit (220), eines Aufwärmstatus des Benutzers entsprechend einem Maß der Blutperfusion, die anhand von Eigenschaften der AC-Komponente, der DC-Komponente oder der Impulsmorphologie des Ausgangssignals (101) ermittelt wurde.

6. Computerprogrammprodukt umfassend einen computerlesbaren Speicher, in dem Computerprogrammcodemittel gespeichert sind, die das Herzfrequenzüberwachungssystem nach Anspruch 1 veranlassen, die Schritte des Verfahrens zum Ermitteln eines Aufwärmstatus eines Benutzers nach Anspruch 5 durchzuführen.

## Revendications

1. Système de surveillance de la fréquence cardiaque, comprenant :
- un capteur de fréquence cardiaque optique (100), comprenant :
- au moins une source de lumière (110) configurée pour générer de la lumière qui est dirigée vers la peau (1000) d'un utilisateur, et
- au moins une unité photodétectrice (120) configurée pour détecter de la lumière et émettre un signal de sortie (101) qui est indicatif d'une absorption ou d'une réflexion de la lumière depuis l'au moins une source de lumière (110) dans ou de la peau (1000) ;
- une unité d'analyse (200) configurée pour déterminer une fréquence cardiaque d'un utilisateur sur la base du signal de sortie (101) du capteur de fréquence cardiaque optique (100) ;
**caractérisé en ce que**
- l'unité d'analyse (200) comprend en outre :
- un analyseur de signal (210) configuré pour analyser au moins l'un d'un composant AC, d'un composant DC et d'une morphologie d'impulsion du signal de sortie (101) ; et
- une unité de détecteur de réchauffement (220) configurée pour déterminer un statut de réchauffement de l'utilisateur selon une mesure de perfusion sanguine déterminée à partir de caractéristiques du composant AC, du composant DC ou de la morphologie d'impulsion du signal de sortie (101).

2. Système de surveillance de la fréquence cardiaque selon la revendication 1, dans lequel les caractéristiques de signal du signal de sortie (101) comprennent une valeur instantanée ou une valeur temporelle moyenne du composant DC, une amplitude instantanée ou une amplitude temporelle moyenne du composant AC, et/ou une morphologie d'impulsion instantanée ou temporelle moyenne.

3. Système de surveillance de la fréquence cardiaque selon la revendication 2, dans lequel l'unité de détecteur de réchauffement (220) est configurée pour comparer les caractéristiques de signal analysées, leurs changements relatifs et/ou absolus dans le temps, à des valeurs seuils pour déterminer si l'utilisateur est déjà réchauffé.

4. Système de surveillance de la fréquence cardiaque selon la revendication 3, comprenant en outre une unité de sortie (300) configurée pour émettre un statut de réchauffement d'un utilisateur.

5. Procédé de détermination d'un statut de réchauffement d'un utilisateur, comprenant les étapes de :
- génération de lumière par une source de lumière (110) et direction de ladite lumière vers la peau (1000) de l'utilisateur ;
- détection de l'absorption ou de la réflexion de la lumière depuis l'au moins une source de lumière (110) dans ou de la peau (1000) par une unité photodétectrice (120) et génération d'un signal de sortie (101) indicatif de celles-ci ;
- l'analyse par un analyseur de signal (210) d'au moins l'un du composant AC, du composant DC et de la morphologie d'impulsion du signal de sortie (101) et la détermination d'une fréquence cardiaque de l'utilisateur sur la base dudit signal de sortie (101) ;
- la détermination par une unité de détecteur de réchauffement (220) d'un statut de réchauffement de l'utilisateur selon une mesure de perfusion sanguine déterminée à partir de caractéristiques du composant AC, du composant DC ou de la morphologie d'impulsion du signal de sortie (101).

6. Produit de programme informatique, comprenant des moyens de codage d'un programme informatique stocké dans une mémoire, pouvant être lu par un ordinateur, pour que le système de surveillance de la fréquence cardiaque selon la revendication 1 réalise les étapes du procédé de détermination d'un statut de réchauffement d'un utilisateur selon la revendication 5.
